# EUROPEAN PATENT APPLICATION

(11) **EP 1 837 027 A1**
(43) Date of publication of application: **26.09.2007**
(21) Application number: 07103802.0
(22) Date of filing: 08.03.2007
(51) Int. Cl.: A61K 31/7048, C07H 17/04, A61P 37/04

(54) **Iridoid glycoside composition**

(30) Priority: 08.03.2006 IN DE06142006
(71) Applicant: Council of Scientific and Industrial Research, New Delhi - 110 001 (IN); Bharat Biotech International Limited, Hyderabad 500 078 (IN)
(72) Inventor: Anamika, Khajuria, 180016, Jammu (IN); Amit, Gupta, 180016, Jammu (IN); Surjeet, Singh, 180016, Jammu (IN); Fayaz, Malik, 180016, Jammu (IN); Jaswant, Singh, 180016, Jammu (IN); Kasturi Lal, Bedi, 180016, Jammu (IN); Avtar, Suri, Krishan, 180016, JAMMU (IN); Kumar, Satti, Naresh, 180016, Jammu (IN); Om Prakash, Suri, 180016, Jammu (IN); Nabi, Qazi, Ghulam, 180016, Jammu (IN); Kannappa, Srinivas, Vellimedu, 500078, Hyderabad (IN); Krishna, Ella, 500078, Hyderabad (IN)
(74) Representative: Portal, Frédéric

(57) **Abstract**

The present invention relates to an adjuvants, particularly to the use of a well-characterized plant based iridoid glycoside adjuvant from plant *Picrorhiza kurroa,* acting as an adjuvant against T -dependent antigen and specifically against HBsAg and typhoid antigens.

The present invention also relates to the method of producing the iridoid glycoside adjuvant and the products utilizing such adjuvants for induction of cellular immunity. The adjuvants may be used alone or with specific antigens. The two antigens used in the study represents HBsAg, a recombinant antigen expressed in Pichia pastoris, and typhoid Vi polysaccharide purified from Salmonella typhi broth. These antigens are studied for their immunogenicity with the adjuvant iridoid glycoside adjuvant.

## Description

### FIELD OF INVENTION

The present invention is in the field of plant based adjuvant and vaccines and use thereof.

### BACKGROUND OF THE INVENTION

*Picrorhiza kurroa* is a well-known herb in the Ayurvedic system of medicine and has traditionally been used to treat disorders of the liver and upper respiratory tract, reduce fevers, and to treat dyspepsia, chronic diarrhea, and scorpion sting. lt is a small perennial herb from the Scrophulariaceae family, found in the Himalayan region growing at elevations of 3,000-5,000 meters. *Picrorhiza kurroa* has a long, creeping rootstock that is bitter in taste, and grows in rock crevices and moist, sandy soil. The leaves of the plant are flat, oval, and sharply serrated. The flowers, which appear June through August, are white or pale purple and borne on a tall spike; manual harvesting of the plant takes place October tnrough December. The active constituents are obtained from the root and rhizomes. The plant is self-regenerating but unregulated over-harvesting has caused it to be threatened to near extinction. Current research on *Picrorhiza kurroa* has focused on its hepatoprotective, anticholestatic, antioxidant, and immune-modulating activity (Atal et al, 1986; Subedi, 2000).
The immunobiological activity was investigated of certain medicinal plants widely used in the Ayurvedic and Unani systems of medicine for treatment of chronic infections and immunological disorders.

Iridoid glycoside adjuvantis a standardized mixture of iridoid glycosides (picroside I and picroside II) isolated from the rhizomes of *P. kurroa* (Kitagawa et al, 1971; Weinges et al, 1972), and its yield from freshly dried plant material is between 3 and 5%. The effect;of a iridoid glycoside adjuvant was studied on delayed type hypersensitivity, humoral responses to sheep red blood cells, skin allograft rejection, and phagocytic activity of the reticuloendothelial system in mice. *Picrorhiza kurroa* was found to be a potent immunostimulant, stimulating both cell-mediated and humoral immunity.
HPLC standardized iridoid glycoside adjuvant tested using both particulate and soluble model antigens including *Candida albicans* and *Salmonella typhimurium.* These significantly stimulate the cellular expression and activation in mice and rats and suppress the fungal and bacterial infections. Iridoid glycoside adjuvant did not produces any intradermal apparent toxicity.iridoid glycoside adjuvant increased the antigen specific IgG response to bovine serum albumin (BSA). The adjuvant effect, which increased the total IgG titre to BSA was more than 80% after two immunization; iridoid glycoside adjuvant when administered as co-adjuvant with muramyl dipeptide (MDP), produced higher antibody titres than those induced with MDP alone.

It was further observed that iridoid glycoside adjuvant significantly stimulated antibody titres, and cell mediated immune response to specific antigens. The effect was superior after the recall dose of antigen with particular reference to antibody titres and DTH reaction. The cellular mediators involved in the expression of cell mediated immunity as observed by way of IL-2 and IFNγ cytokines, are known for CTL activity aimed at killing infected cells and expressing abnormal antigens. Thus the proposed entity iridoid glycoside adjuvant has projected them to be strong candidates for evaluation as immunoadjuvant for vaccines.

Wide varieties of antigens stimulate the production of antibodies in animals and confer protection against subsequent infections. However, some antigens stimulate only a mild or ineffective immune response, while some are unable to stimulate an effective immune response.

The immunogencity of weak antigen is often enhanced by simultaneous administratio of antigen with an adjuvant, which is a substance that may or may not be immunogenic when administered alone but it will induce a state of immunity, may it be systemic or mucosal for an antigen. Unfortunately many immunoadjuvants are toxic unsafe and are only useful for animals, not human vaccination. Preferred adjuvants are substance that are not mitogenic or very toxic, but potentiate and focus the immune response to vaccine, Adjuvants often contain immunomodulators, which induce the production of cytokine cascade and result in augmented immune response.

Iridoid glycoside adjuvant glycosides of plant *P.kurroa* has been shown to act as adjuvant so as to affect immunoadjuvant effects on peripheral blood mononuclear cells (PBMC) *in-vitro* stimulated with sub immunogenic dose of HBsAg. This lymphocyte activation appears likely that the rapid immune activation in response to iridoid glycoside adjuvant may have evolved as one component of the innate immune defense mechanisms that recognize structural patterns specific to immune-stimulation.
Iridoid glycoside adjuvant induces proliferatio of almost all (>92%) B cells and increases immunoglobulin (Ig) secretion. The iridoid glycoside adjuvant led to an increase in the levels of the cytokine IL-4 & IL-10 and might enhance the humoral immune response. This B cell activation by iridoid glycoside adjuvant is T cell dependent and antigen non-specific. However, B cell activation by low concentratios of iridoid glycoside adjuvant has strong synergy with signals delivered through the B cell antigen receptor for both B cell proliferatio and Ig secretion. This strong synergy between the B cell signaling pathways triggered through the B cell antigen receptor and by iridoid glycoside adjuvant promotes antigen specific immune responses. In addition to its direct effects on B cells In addition, iridoid glycoside adjuvant directly activates monocytes, macrophages, and dendritic cells to secrete a variety of cytokines, including high levels of IL-12. These cytokines stimulate natural killer (NK) cells to secrete gamma-interferon (IFN-gamma) and have increased lytic activity (Klinman et al, 1996; Akbar, 1999; Ballas et al., 1996). Overall, iridoid glycoside adjuvant induces a Th1 like pattern of cytokine production dominated by IL-12 and IFN-.gamma with secretion of Th2 cytokines also.
To start with adjuvant effect of iridoid glycoside adjuvant, a specific antigen Hepatitis B virus has been evaluated. Since (HBV) posses a serious worldwide health problem. The current HBV vaccines are subunit vaccines containing particles of HBV envelope protein(s), which include several B and T cell epitopes known collectively as HBV surface antigen (HBsAg). The HBsAg particles may be purified from the plasma of chronically infected individuals or more commonly are produced by recombinant DNA technology. These vaccines induce antibodies against HBsAg (anti-HBsAg), which confer protection if present in titers of at least 10 milli-international Units per milliliter (mlU/ml) (Brunel, 1999). The current subunit vaccines, which contain alum (a Th2 adjuvant), are safe and generally efficacious. They, however, fail to meet all current vaccination needs. For example, early vaccination of infants born to chronically infected mothers, as well as people in endemic areas, drastically reduces the rate of infection, but a significant proportion of population will still become chronically infected themselves (Shlomai, 2003). This could possibly be reduced if high titers of anti-HBsAg antibodies could be induced earlier and if there were HBV-specific CTL (Bohm, 1998). In addition, there are certain individuals who fail to respond (non-responders) or do not attain protective levels of immunity (hypo-responders). Finally, there is an urgent need for an effective treatment for the estimated 350 million chronic carriers of HBV and a therapeutic vaccine could meet this need.
Secondly, the immunogenicity of Vi polysaccharide typhoid antigen is also evaluated.
Typhoid (cloudy) fever is a systemic infection, caused mainly by *Salmonella typhi* found only in man. It is characterized by a continuous fever for 3-4 weeks, relative bradycardia, with involvement of lymphoid tissue and considerable constitutional symptoms. In western countries, the disease has been brought very close to eradication levels (a).

Each year, the world over, there are at least 13-17 million cases of typhoid fever, resulting in 600,000 deaths. 80% of these cases and deaths occur in Asia alone,

Antibiotics resistance, particularly emergence of multidrug resistant (MDR) strains among *Salmonellae* is also a rising concern and has recently been linked to antibiotic use in livestock. Many *S typhi* strains contain plasmids encoding resistance to chloramphenicol, ampicillin and co-trimoxazole, the antibiotics that have long been used to treat enteric fever. In addition, resistance to ciprofloxacin also called nalidixic-acid resistant *S typhi* (NARST) strain either chromosomally or plasmids encoded, has been observed in Asia.

These studies as well as the efficacy of the antigen is formulated with different doses of iridoid glycoside adjuvant and the compared with the conventional typhoid vaccine which is non adjuvanted and comprises of only 25 mcg /human dose.

Akbar SMF, Abe M, Masumoto TK, Horiike N, Onji M, 1999. Mechanism of action of vaccine therapy in murine hepatitis B virus carriers: vaccine-induced activation of antigen presenting dendritic cells. Hepatology, 30, 755 - 764.

Atal CK, Sharma ML, Kaul A, Khajuria A, 1986. Immunomodulating agents of plant origin. I: preliminary screening. J Ethnopharmacol 18, 133-141,

Ballas ZK, Rasmussen WL, Krieg AM, 1996. Induction of NK activity in murine and human cells by CpG motifs in oligodeoxynucleotides and bacterial DNA Journal of Immunology 9 (1), 1840 - 1845.

Bohm W, Mertens T, Schirmbeck R, Reimann J, 1998. Routes of plasmid DNA vaccination that prime murine humoral and cellular immune responses. Vaccine, 16 (9-10), 949-954.

Bres EM, Payette PJ, Mancini M, Tiollais P, Davis HL, Michel ML, 2001. CpG oligodeoxynucleotides with hepatitis B surface antigen (HBsAg) for vaccination in HBsAg-Transgenic mice. Journal of virology, 75 (14), 6482 - 6491.

Brunel F, Darbouret A, Ronco J, 1999. Cationic lipid DC-chol induces an improved and balanced immunity able to overcome the unresponsiveness to the hepatitis B vaccine. Vaccine, 17 (17), 2192 - 2203.

Chander R, Kapoor NK, Dhawan BN, 1992. Picroliv, picroside-1 and kutkoside from Picrorhiza kurroa are scavengers of superoxide anions. Biochem Pharmacol, 44, 180 - 183.
Gargiulo F, Monti E, Caruso A, Manca N, Martinelli F, Rango CD, Flamminio G, Gao J, Preti A, Turano A, 1993. High-titre antibodies to a foreign epitope elicited by affinity-purified hybrid LamB proteins. Vaccine 11 (11), 1093-1096.

Kitagawa I, Hino K, Nishimura T, Iwata E, Yosioka I, 1971. On the constituents of Picrorhiza kurroa. The structure of picroside I, Chem Pharm Bull 19, 2534-2544.

Klinman DM, Yi AK, Beaucage SL, Conover J, Kreig AM, 1996. CpG motifs presents in bacterial DNA rapidly induce lymphocytes to secrete interleukin 6, interleukin 12 and interferon y. Molecular Medicine Today, 2 (6), 233.

Li JX, Li P, Tezuka Y, Namba T, Kadota S, 1998. Three phenylethanoid glycosides and an iridoid glycoside from Picrorhiza Scrophulariiflora. Journal phytochemistry, 48 (3), 537 - 542.

Rivera E, Pettersson, FE, Inganas M, Paulie S, Gronvik KO, 2005. The Rb1 bioactive fraction (adjuvant) of ginseng elicits a balanced Th1 and Th2 immune response. Vaccine, 23 (46-47), 5411-5419.

Shlomai A, Shaul Y, 2003. Inhibition of hepatitis B virus expression and replication by RNA interference. Hepatology, 37 (4), 764 - 770.

Subedi BP, 2000. Plant profile: Kutki (Picrorhiza scrophulariiflora), Himalayan Bioresources 4, 14-15.

Weinges K, Kloss P, Henkels WD, 1972. Picroside 11, ein neues 6-vanilloyl-cataipolaus Picrorhiza kurroa Royle und Benth Liebigs, Ann Chem 759, 173-182.
Chen JZ, Zhu HH, Liu KZ, Chen Z, 2004. Enhancing cellular immune response to HBV M DNA vaccine in mice by codelivery of interteukin-18 recombinant. Chen et al./J Zhejiang Univ SCI 5 (4), 467 - 471.
Lt Gen SP Kalra AVSM Bar*, Lt Col N Naithani+, Col SR Mehta VSM#, Sqn Ldr AJ Swamy MJAFI 2003; 59 : 130-135

### OBJECTS OF THE INVENTION

The main object of the present invention is to provide a plant based vaccine adjuvant.

Another object of the present invention is to provide an iridoid glycoside adjuvant from plant *Picrorhiza kurroa* comprising PKI and PKII.

Another object of the present invention is to provide a process for preparatio of the said (adjuvant).

Yet another object of the invention is to provide a vaccine formulation comprising of the adjuvant and antigen optionally along with pharmaceutically acceptable carriers, diluents.

Yet another object of the present invention is to provide a vaccine formulation comprising of combination of adjuvant and antigen with optionally pharmaceutically acceptable carriers, diluents.

Another object of the present invention is to provide a vaccine formulation useful for modulation of cytokine levels.

Another object of the present invention is to provide a vaccine formulation useful for activation uf cells of immune system.

### SUMMARY OF THE INVENTION

The present invention relates to an adjuvant, particularly to an iridoid glycoside adjuvant obtained from plant *Picrorhiza kurroa,* acting as an adjuvant against T -dependent antigen and specifically against HBsAg and typhoid antigens.

The present invention also relates to the method of producing the said adjuvant and the products utilizing such adjuvants for induction of cellular immunity. The adjuvants may be used alone or with specific antigens.
Accordingly the present invention provides a synergistic vaccine composition comprising iridoid glycosides Picroside I and Picroside-II represented by following formula, wherein the ratio of PKI ranging between 0.80-1.25 and PK II ranging between 1.60-2.50. 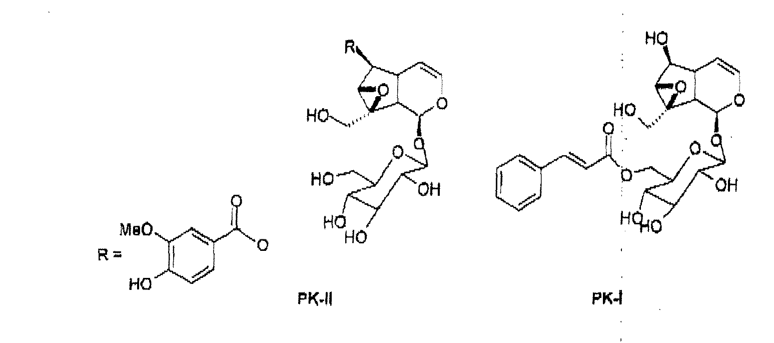 In an embodiment of the present invention, the said iridoid glycosides is obtained from the plant *Picrorhiza kurroa*
In yet another embodiment of the present invention, the shelf- life of the adjuvant is 175 weeks.

In another embodiment of the present invention the said adjuvant exhibits potency at microgram quantities and integrates easily with HBsAg formulation.

In a still another embodiment the said adjuvant is required in reduced dose as compared to the other adjuvant.

In a further embodiment the said adjuvant is safe, well tolerated and immunogenic promoting more rapid protection against hepatitis B infection in comparison with others adjuvants (CFA & MDP).

In yet another embodiment the said adjuvant increases levels of both the cytokine (Th1 and Th2) whereas MDP and FCA induced only Th1 type of immune response compared with the, MDP and FCA when co-administered with HBsAg.

In another embodiment the said adjuvant favours an enhancement in the IgG titers against HBsAg antigen containing alum.

In another embodiment the said adjuvant has the potential ability to increase total vaccine HBsAg specific antibody response and T cell response at 2.5 µg/ml.

In another embodiment the said adjuvant when administered in combination of alum together with HBsAg generates higher protective serum IgG antibody response, proving synergetic effect.

In another embodiment the said adjuvant appears to increase potency with relatively small quantities of antigen and exhibit synergy with other adjuvants because of the ability to improve the body's immune response to very low doses of antigen.

In still another embodiment the said adjuvant together with antigen HBsAg reduces the dose of antigen from 20µg (standard vaccine containing 20 µg + alum) to 15 µg (iridoid glycoside adjuvant+ HBsAg).

In a further embodiment the said adjuvant together with antigen HBsAg promotes CD8 population observed on day 15 and 28 in mice as compared with alum co-administered with HBsAg, which is poorly elicited CD8 population.

In another embodiment the said adjuvant together with HBsAg increases specific lgG1 and IgG2a response in mice as compared with alum co-administered with HBsAg, which increases only IgG1 response but poorly elicited IgG2a response.

In another embodiment the efficacy of the said adjuvant together with variable doses of antigen HBsAg on serum immunoglobulins, is higher as compared to alum containing HBsAg .

In another embodiment the effect of 2.5 µg of said adjuvant together with antigen HBsAg (20 µg) on serum immunoglobulins, is up to 1:4500 in comparison with alum containing Bag.

ln another embodiment the effect of said adjuvant together with antigen HBsAg provides enhanced lL-2, IL-12, IFN-gamma, TNF-alpha secreted by Th1 cells and lL

In another embodiment the above said adjuvant is useful for activation of cells of immune system.

An embodiment, of the invention is a vaccine formulation comprising of iridoid glycoside adjuvant, an antigen optionally along with one or more other adjuvants and pharmaceutically acceptable additives.

In another embodiment the other adjuvants are selected from the group comprising alum, monophosphoryl lipid, CompleteFreund's Adjuvant, Muramyl dipeptide.

In another embodiment the antigen is selected from the group comprising HBsAg, typhoid Vi polysaccharide peptides, polypeptides, cells, cell extracts, polysaccharides, polysaccharide conjugates, lipids, glycolipids, carbohydrates, Virus particles and recombinant proteins, a crude, purified or recombinant form, peptide mimics of polysaccharides, tumour antigen, allergen, bacteria, fungus, protozoa, parasites.

In another embodiment of the present invention the ratio of iridoid glycoside adjuvant:
alum: antigen ranges from of 0.125:14.5:1.

In another embodiment the said formulation can be injectible in bolus or continuous infusion.

In another embodiment the effective dose (0.312 µg -40.0 µg) of said formulation is administered to a subject by injection.

ln another embodiment the said formulation is administered by oral, intradermal, intraperitoneal, intramuscular route.

In another embodiment the said formulation is useful for activation of cells of immune system.

In another embodiment the said formulation is useful for modulation of cytokine levels.

In another embodiment the said iridoid glycoside adjuvant is useful for modulation of Th1 and Th 2 cytokines.

An embodiment of the present invention is a vaccine formulation comprising recombinant hepatitis B surface antigen protein, iridoid glycoside adjuvant, optionally along with alum and other pharmaceutically acceptable additives.

In another embodiment of the invention the said formulation consists of iridoid glycoside adjuvant, alum and recombinant hepatitis B surface antigen protein in a ratio of 0.125:72.5:1.

In yet another embodiment of the present invention the formulation consists of recombinant hepatitis B surface antigen protein and iridoid glycoside adjuvant.

In still another embodiment of the present invention the ratio of iridoid glycoside adjuvant ranges between 1 to 128 and ratio of hepatitis B surface antigen ranges between 1 to 10.

An embodiment of the present invention is a vaccine formulation comprising typhoid Vi polysaccharide, iridoid glycoside adjuvant and optionally alum.

In an embodiment of the present invention the said formulation comprises iridoid glycoside adjuvant, alum and typhoid Vi polysaccharide in ratio a ranging D.1:58:1.

An embodiment of the invention is a method of immunization of a subject with vaccine formulation wherein an effective dose (0.312-40.0 µg) of said formulation is administered to a subject by injection.

In an embodiment of the invention the said formulation is administered by oral, intradermal, intraperitoneal, intramuscular route.

In another embodiment of the invention the said formulation is administered as unit doses and booster doses.

In yet another embodiment of the invention the said subject is mammal, including human being.

An embodiment of the present invention is a process for preparatio of vaccine adjuvant comprising the steps:
a) extracting the powdered dried roots of P. Kurroa with an organic solvent selected form a group consisting of dichloroethane methane petroleum ether, dichloroethane to obtain the marc,
b) extracting the said marc with an organic solvent up to a refluxing temperature,
c) separating the extract form the suspended particles and concentrating to obtain the residue,
d) extracting the residue obtained in step (c) with chloroform and ethyl acetate and extracts are discarded and the residue is dissolved in ethanol or methanol,
e) cooling the ethanolic/ methanolic solution obtained in step (d), and adding diethyl ether till turbidity persists,
f) recovering solid by filtratio and decolorizing using activated charcoal as decolonizing agent to obtain the mixture of picroside I & II.
g) standerdising the above said mixture of picroside I & II by HPLC fingerprinting,

### BRIEF DESCRIPTION OF THE DRAWINGS

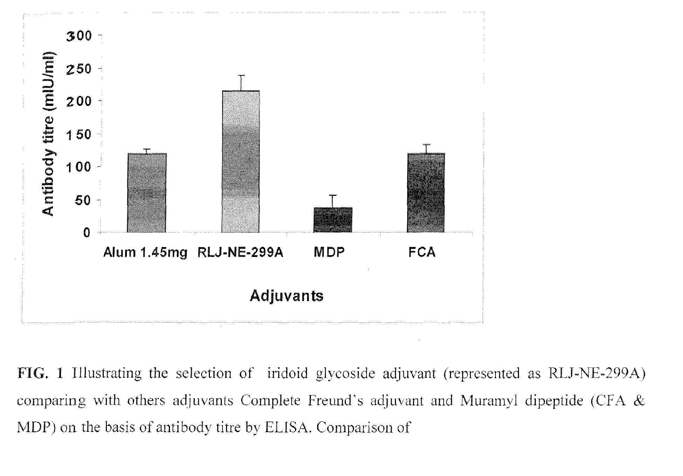

**FIG. 1** Comparison of the iridoid glycoside adjuvant with other adjuvants i.e. Complete Freund's adjuvant and Muramyl dipeptide (CFA & MDP) on the basis of antibody titre by ELISA.

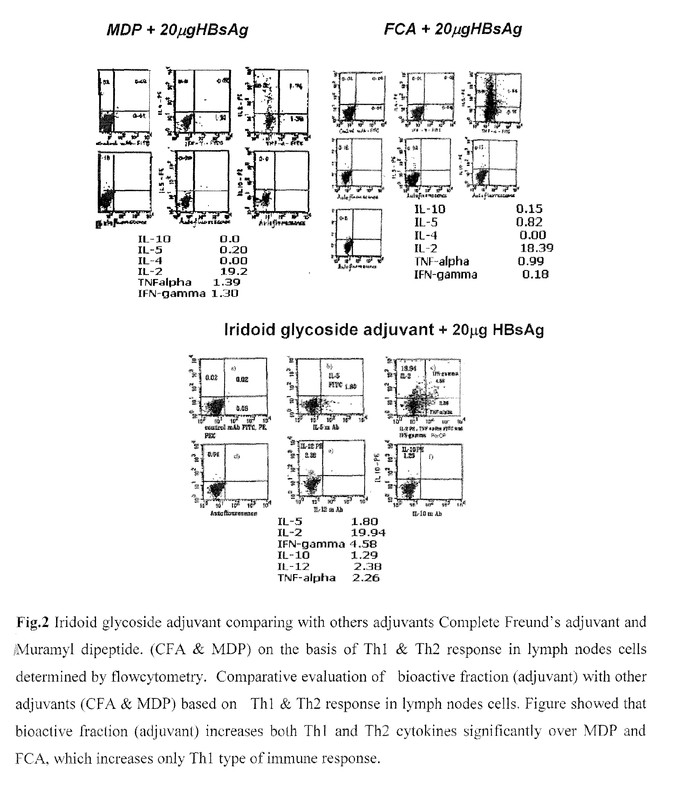

**Fig.2** Comparison of iridoid glycoside adjuvant with other adjuvants i.e. Complete Freund's adjuvant and Muramyl dipeptide (CFA & MDP) on the basis of Th1 & Th2 response in lymph nodes cells determined by flowcytometry.

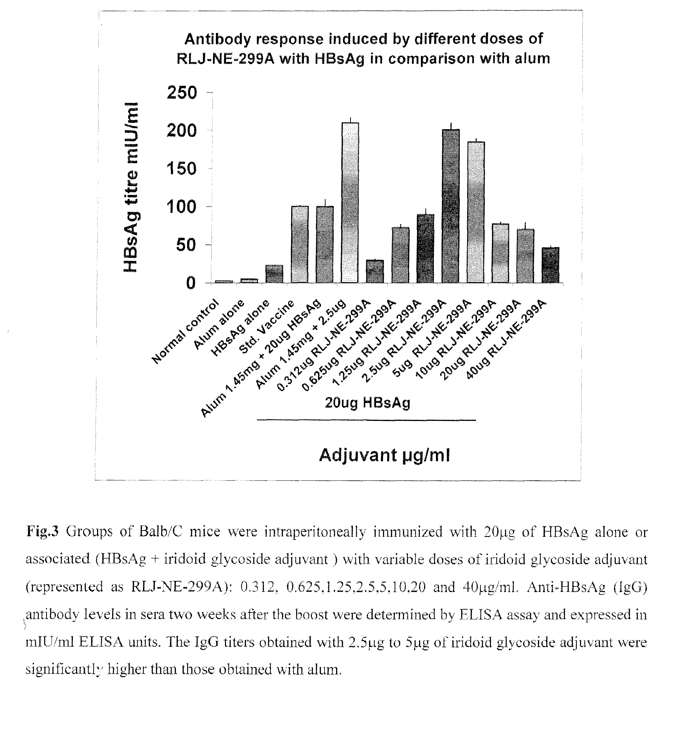

**FIG. 3** Graph illustrating humoral responses in Balb/c mice immunized with 1ml of formulation of HBsAg protein 20µg with or without alum and with variable doses 0.312, 0.625. 1.25, 2.5, 5, 10, 20, 40 µg/ml of iridoid glycoside adjuvant added. Each point represents the group mean for anti-HBsAg titers (total IgG) as determined by end-point dilution ELISA assay. Results are expressed on the basis of three sets of experiments.

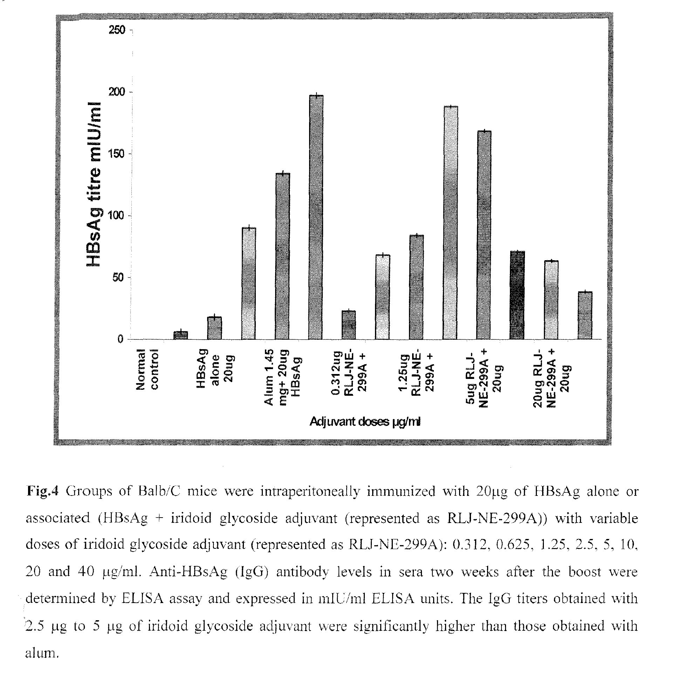

**Fig. 4** Graph comparing the IgG antibody response obtained by immunization with iridoid glycoside adjuvant and other adjuvants.

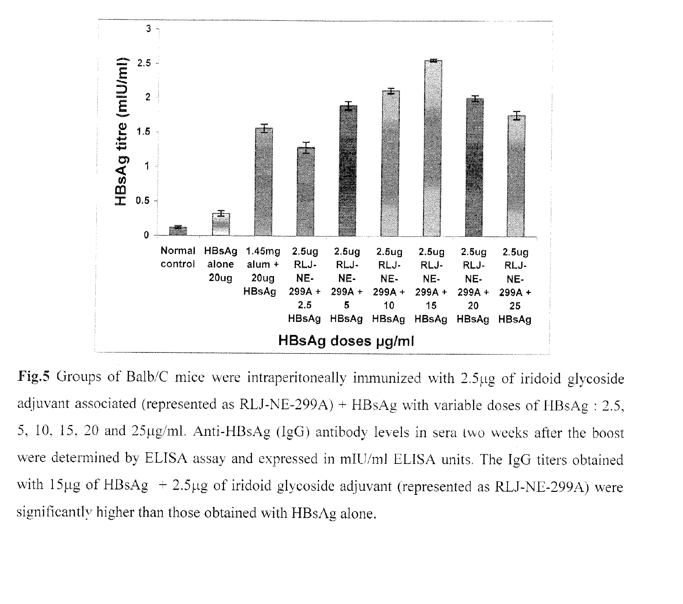

**FIG. 5** Graph illustrating humoral responses in Balb/C mice immunized with 1 ml of selective dose of adjuvant 2.5µg of Iridoid glycoside adjuvant and variable doses of HBsAg 2.5,5,10,15,20,25µg/ml protein without alum. Each point represents the group mean for anti-HBsAg titer (total IgG) as determined by end-point dilution ELISA assay.

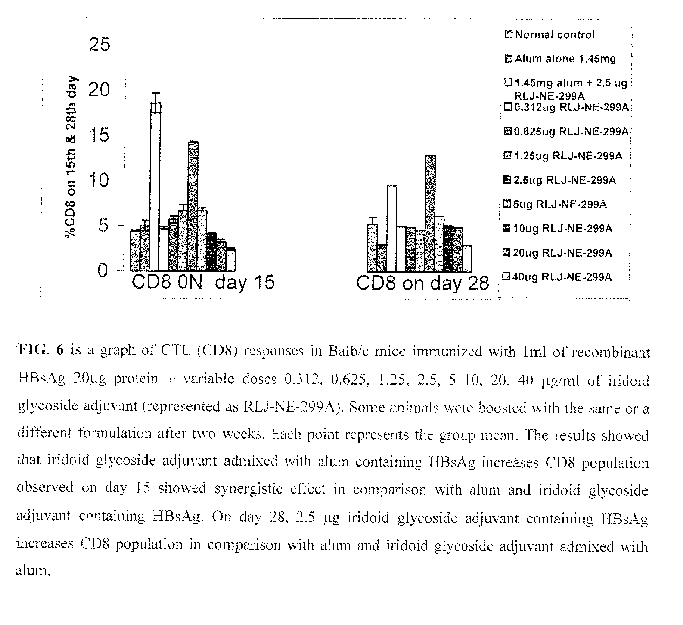

**FIG. 6** Graph of CTL (CD8) responses in Balb/c mice immunized with 1ml of recombinant HBsAg 20 µg protein + variable doses 0.312, 0.625, 1.25, 2.5, 5 10, 20, 40 µg/ml of Iridoid glycoside adjuvant, Some animals were boosted with the same or a different formulation (alum) after two weeks. Each point represents the group mean.

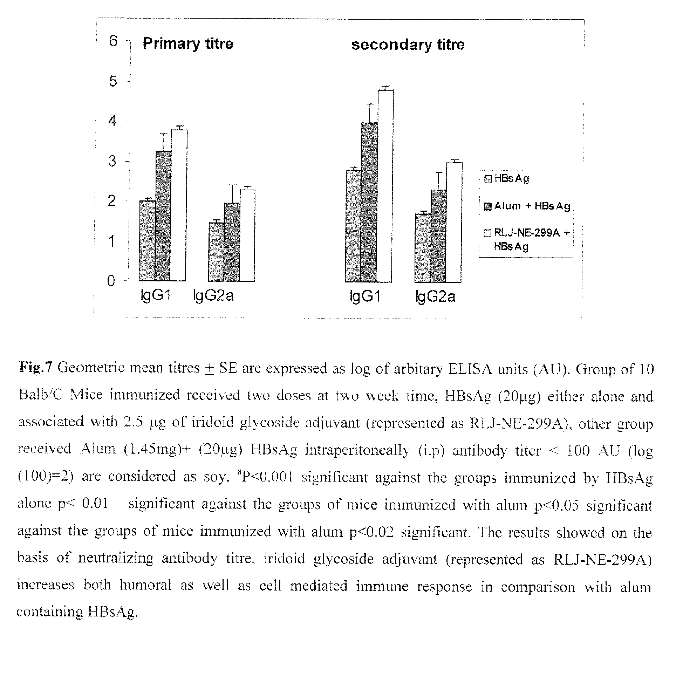

**FIG. 7.** Bar graph depicting the amount of total IgG (lgG1 and IgG2a) end-point ELISA titer produced at 4 weeks in Balb/C mice immunized with 20µg of HBsAg + 2.5 µg of Iridoid glycoside adjuvant, indicating a more Th1 as well as Th2 response.
**FIG. 8**.Graph representing humoral antibody titer with variable doses of HBsAg (2.5-15µg with constant dose of Iridoid glycoside adjuvant adjuvant 2.5 µg and other group of animals receiving HBsAg antigen alone or 1.45 mg alum + 20 µg HBsAg to see the effect on antibody titre by flowcytometry.

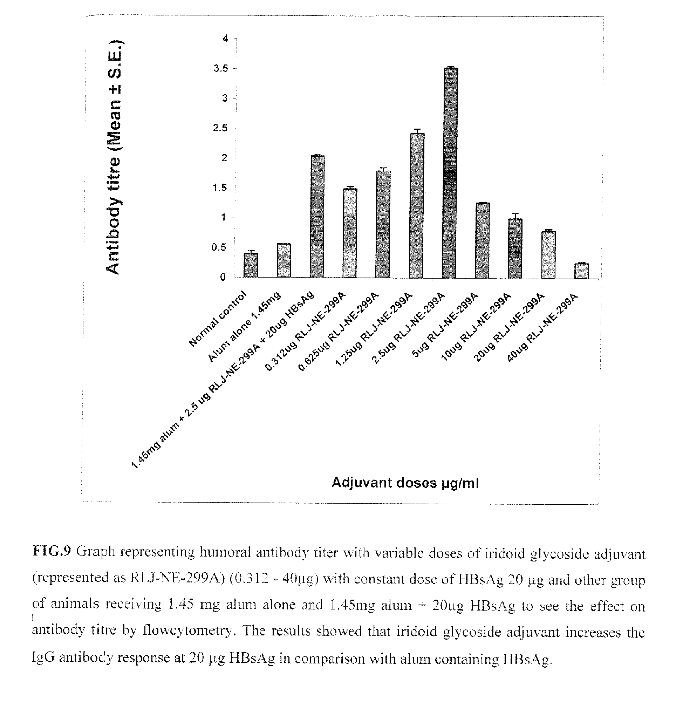

**FIG. 9**.Graph representing humoral antibody titer with variable doses of Iridoid glycoside adjuvant adjuvant (0.312-40µg) with constant dose of HBsAg 20 µg and other group of animals receiving 1.45mg alum alone and 1,45mg alum + 20µg HBsAg to see the effect on antibody titre by flowcytometry.

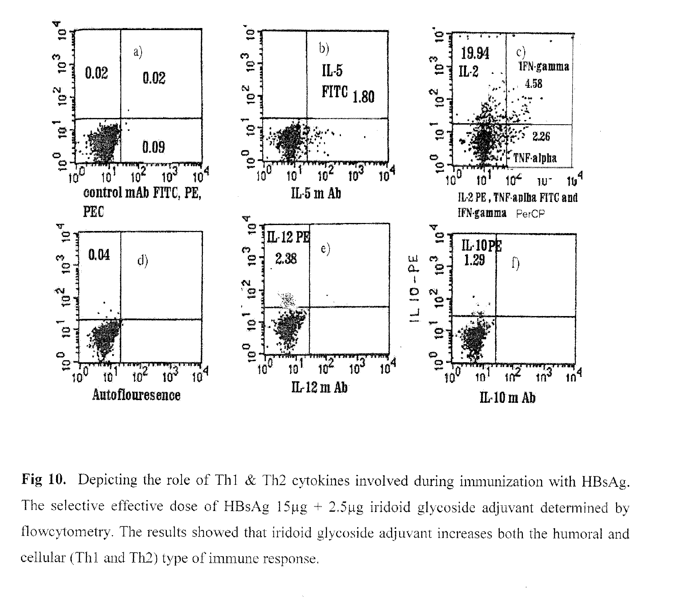

**Fig 10**. Depicting the role of Th1 & Th2 cytokines involved during immunization with HBsAg. The selective effective dose of HBsAg 15 µg + 2.5 µg Iridoid glycoside adjuvant determined by flowcytometry.

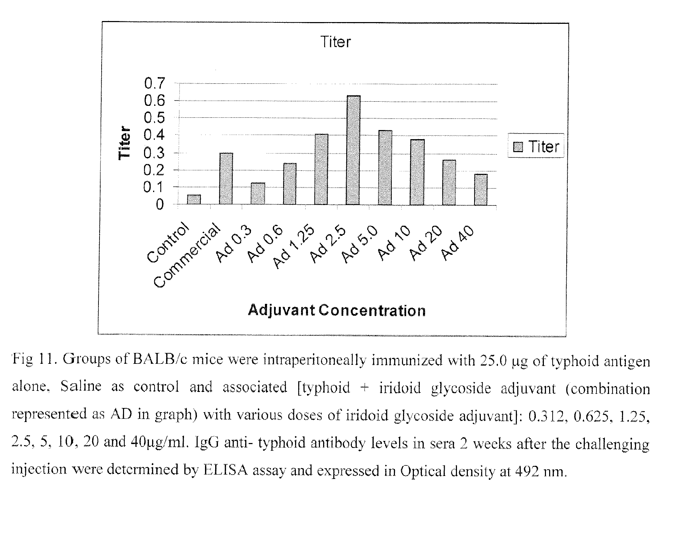

**Fig 11.** Groups of BALB/c mice were intraperitoneally immunized with 25.0 µg of typhoid antigen alone, Saline as control and associated (typhoid +Iridoid glycoside adjuvant) with various doses of Iridoid glycoside adjuvant: 0.312, 0.625, 1.25, 2.5, 5, 10, 20 and 40µg/ml. IgG anti- typhoid antibody levels in sera 2 weeks after the challenging injection were determined by ELISA assay and expressed in Optical density at 492 nm.

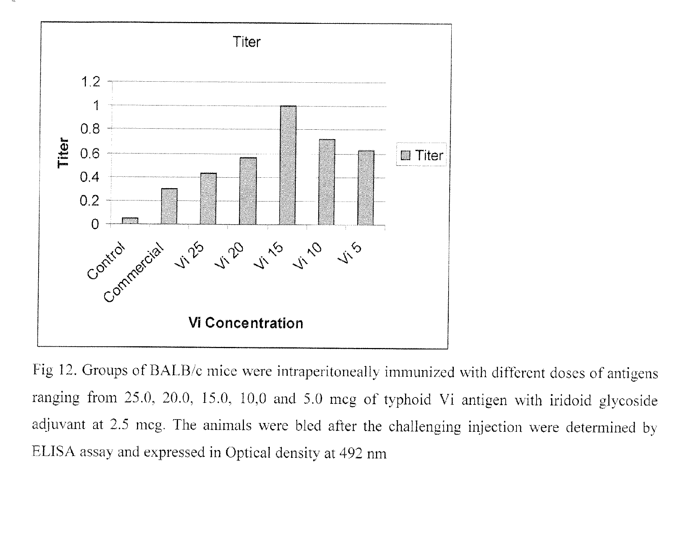

**Fig 12.** Groups of BALB/c mice were intraperitoneally immunized with different doses of antigens ranging from 25.0, 20.0, 15.0, 10,0 and 5.0 mcg of typhoid antigen with iridoid glycoside adjuvant at 2,5 mcg. The animals were bled after the challenging injection were determined by ELISA assay and expressed in Optical density at 492 nm

**Table 1.** Effect of different doses of Iridoid glycoside adjuvant on CD4 and CD8 population on splenocytes determined by flowcytometry
**Table 2.** Effect of Iridoid glycoside adjuvant on T (lFN gamma) and B (lL-4) cell secreted cytokines by ELISA

### DETAILED DESCRIPTION OF THE INVENTION

The present invention relates to a plant based iridoid glycoside adjuvants, obatined from plant *Picrorhiza kurroa,* acting as an adjuvant T dependent antigen and specifically against HBsAg and typhoid antigens.

The present invention also relates to the method of producing the iridoid glycoside adjuvant and the products utilizing such adjuvants for induction of cellular immunity. The adjuvants may be used alone or with specific antigens.

The two antigens used in the study represent HBsAg, a recombinant antigen expressed in *Pichia pastoris,* and typhoid Vi polysaccharide purified from *Salmonella typhi.*

These antigens are studied for their immunogenicity with the iridoid glycoside adjuvant.

The present invention relates to methods and products for inducing an immune response by the use of a chemically characterized plant based iridoid glycoside adjuvant acting as strong vaccine adjuvant. The invention is useful in one aspect as a method of inducing an antigen specific immune response. The method includes the steps of administering iridoid glycoside adjuvant in order to induce an antigen specific immune response. An antigen HBsAg with conventional adjuvant namely alum, a combination of adjuvant iridoid glycoside adjuvant and HBsAg, unadjuvanted Vi polysaccharide and finally a combination of iridoid glycoside adjuvant formulated with Vi polysaccharide are administered in an effective-amount for inducing a synergistic adjuvant response.

The iridoid glycoside adjuvant can be administered with specific and non-specific antigen. Primarily, the combination of iridoid glycoside adjuvant -antigen HBsAg may be administered with priming dose of antigen and adjuvant. In another set of animals groups is administered with a boost dose of antigen.

The antigen may be any type. For example, the antigen may be selected from the group consisting of peptides, polypeptides, cells, cell extracts, polysaccharides polysaccharide conjugates, lipids, glycolipids, carbohydrates, Virus particles and recombinant proteins. Antigens may be given in a crude, purified or recombinant form and polypeptide/peptide antigens, including peptide mimics of polysaccharides, may also be encoded within nucleic acids. Antigens may be derived from an infectious pathogen such as a virus, bacterium, fungus or parasite, or the antigen may be a tumor antigen, or the antigen may be an allergen.

According to another aspect of the invention a method of inducing a Th1 immune response is included The method includes the step of administering antigen adjuvant combination in order to induce a Th1 immune response, wherein the combination of adjuvants is administered in an effective amount for inducing a Th1 immune response. The combination of adjuvants is either administered simultaneously or sequentially. Combination of adjuvants is administered in an effective amount for inducing a synergistic Th1 immune response.

According to other aspects the invention includes a method for immunizing animals (Balb/C mice) with a priming dose of antigen HBsAg and iridoid glycoside adjuvant before the boost dose. In other group of animals a boost dose of antigen is given, in order to induce a Th1 innate immune response. For longer-term protection, these adjuvants may be administered more than once. The invention in other aspects include a method of inducing a non-antigen-specific Th1-type immune response, including Th1 cytokines such as lL-12 and IFN-.gamma, for temporary protection against various pathogens including viruses, bacteria, parasites and fungi.

According to other aspects the invention includes a method for immunizing animal (Balb C mice) with different doses of antigen and adjuvants to establish the most effective combination of antigen and adjuvant which can elicit the highest antibody titre.

The invention in one aspect is based on the discovery that the formulation containing combinations of iridoid glycoside adjuvant and alum synergistically enhance immune responses to a given antigen HBsAg.

It has been discovered according to the invention that the combination of *iridoid glycoside adjuvant,* MPL and alum or other adjuvants results in a synergistic immune response. Compared with the recombinant hepatitis B surface antigen (HBsAg) protein vaccine alone, formulation with alum increases the level of antibodies in mice against HBsAg (anti-HBsAg) to 10 fold whereas addition of *iridoid glycoside adjuvant* increase them 18 fold. When *iridoid glycoside adjuvant* and alum are used together, a 500-1000 times higher level of anti-HBsAg titre was observed, indicating a strong synergistic response. Additionally, it was found according to the invention that immunization with HBsAg and alum resulted in a strong Th2-type response with almost all IgG being of the IgG1 isotype. *iridoid glycoside adjuvant* induced a high proportion of IgG2a, indicative of a Th1-type response. Furthermore, it was discovered according to the invention that in Balb/C mice responses were induced by HBsAg with alum and *iridoid glycoside adjuvant* but not with alum or *iridoid glycoside adjuvant* alone. The antibodies produced with *iridoid glycoside adjuvant* were predominantly of the IgG2a isotype, indicating a strong Th1-type response. This is remarkable considering the strong Th2 bias of the neonatal immune system and the known difficulty in inducing Th1 responses at such a young age. Th1 responses are preferable in some instances since they are a associated with IgG2a antibodies that have better neutralization and opsonization capabilities than Th2-type antibodies. As well, Th1 responses are associated with cytotoxic T lymphocytes (CTL) that can attack and kill virus-infected cells (Rivera, 2005). Indeed, iridoid glycoside adjuvant, alone or in combination with HBsAg induced good CTL activity in both adult and neonatal mice. These studies demonstrate that the addition of *iridoid glycoside adjuvant* to protein or DNA vaccines in combination with other adjuvants is a valid new adjuvant approach to improve efficacy.

Thus in one aspect the invention is a method of inducing an antigen specific immune response in a subject. The method includes the step of administering to the subject order to induce an antigen specific immune response an antigen HBsAg, adjuvant iridoid glycoside adjuvant and a combination of *iridoid* glycoside *adjuvant* and alum), and wherein the combination of *iridoid glycoside adjuvant* and alum is administered in an effective amount for inducing a synergistic adjuvant response.

Many types of infectious pathogens do not have any effective treatments and chronic presence of the pathogen can result in significant damage. For instance, the HBV virus is itself non-pathogenic but with chronic infection the partially developed immune response causes inflammatory changes that eventually leads to cirrhosis and increased risk of hepatocellular carcinoma. An estimated one million people die each year from HBV-related liver disease. Persistent HBV infection of the liver results when acute infection fails to launch an appropriate immune response to clear the virus. Such chronic carriers have circulating HBsAg "e" soluble form of the HBV core antigen (HBsAg) without specific immunity. It is thought that the absence of HBV-specific T-cells, including CTL may contribute to the establishment and maintenance of the chronic carrier state. Indeed, many previously infected individuals, even years after clinical and serological recovery, have traces of HBV in their blood and HBV-specific CTL that express activation markers indicative of recent contact with antigen (zhong, 2004). These results suggest that sterilizing immunity may not occur after HBV infection and that chronic activation of HBV-specific CD4+ and CD8+ T-cells is responsible for keeping the virus under control. There is currently no cure for the HBV chronic infection. Interferon is used currently but this cures only 10-20% of treated individuals (Gargiulo, 1993). Anti-viral drugs (e.g., lamivudine) can reduce circulating virus to undetectable levels, however these return to pretreatment levels if the drug is stopped. Each of these types of treatment is also expensive and has certain undesirable side-effects. Thus the synergistic combination of adjuvants, which induces potent Th1 responses, including CTL, is useful for treating a subject having an infection such as HBV.

*Iridoid glycoside adjuvant* is an investigational adjuvant that causes activation of the cells of the immune system i.e. B and T cells. lt may, for instance, *iridoid glycoside adjuvant* causes an immune cell to release cytokines secreted by T helper cells (Th1 and Th2). This class of adjuvants includes but is not limited to glycosides from the root of the *P.kurroa,* such as Iridoid glycoside adjuvant, peak with HPLC *iridoid glycoside adjuvant* action.
Adjuvants that create a depot effect and stimulate the immune system" are those compounds which have both of the above- identified functions. An "antigen" as used herein is a molecule capable of provoking an immune response. Antigens include but are not limited to peptides, polypeptides, cells, cell extracts, polysaccharides, polysaccharide conjugates, lipids, glycoliopids and carbohydrates. Antigens may be given in a crude, purified or recombinant form and polypeptide/peptide antigens, including peptide mimics of polysaccharides, may also be encoded within nucleic. The term antigen broadly includes any type of molecule, which is recognized by a host immune system as being foreign. Antigens include but are not limited to cancer antigens, microbial antigens, and allergens,

The invention further provides a method of modulating the level of a cytokine. The term "modulate" envisions the suppression of expression of a particular cytokine when lower levels are desired, or augmentation of the expression of a particular cytokine when higher levels are desired. Modulation of a particular cytokine can occur locally or systemically. *Iridoid glycoside adjuvant* can directly activate macrophages and dendritic cells to secrete cytokines. No direct activation of proliferatio or cytokine secretion by highly purified T cells has been found, although they are induced to secrete cytokines by cytokines secreted from macrophages and may be costimulated through the T cell Receptor. Cytokine profiles determine T cell regulatory and effector functions in immune responses. In general, Th1-type cytokines are induced, thus the immunostimulatory nucleic acids promote a Th1 type antigen-specific immune response including cytotoxic T-cells.
Cytokines also play a role in directing the T cell response. Helper (CD4+) T cells orchestrate the immune response of mammals through production of soluble factors that act on other immune system cells, including B and other T cells. Most mature CD4+ T helper cells express one of two cytokine profiles: Th1 or Th2. Th1 cells secrete IL-2, IFN-gamma. GM-CSF and high levels of TNF-alpha, Th2 cells express IL-4, IL-5, IL-6, IL-10, IL-13, GM-CSF and low levels of TNF-alpha. The Th1 subset promotes both cell-mediated immunity, and humoral immunity that is characterized by immunoglobulin class switching to IgG2a in mice. Th1 responses may also be associated with delayed-type hypersensitivity and autoimmune disease. The Th2 subset induces primarily humoral immunity and induces class switching to IgG1 and IgE. The antibody isotypes associated with Th1 responses generally have good neutralizing and opsonizing capabilities whereas those associated with Th2 responses are associated more with allergic responses.

Several factors have been shown to influence commitment to Th1 or Th2 profiles, The best-characterized regulators are cytokines IL-12 and IFN-gamma are positive Th1 and negative Th2 regulators. IL-12 promotes IFN-gamma production; IFN-gamma provides positive feedback for IL-12, IL-4 and IL-10 appear to be required for the establishment of the Th2 cytokine profile and to down-regulate Th1 cytokine production; the effects of IL-4 are in some cases dominant over those of IL-12; IL-13 was shown to inhibit expression of inflammatory cytokines, including IL-12 and TNF-.alpha by LPS-induced monocytes, in a way similar to IL-4. The IL-12 p40 homodimer binds to the IL-12 receptor and may antagonize IL-12 biological activity; thus it blocks the pro-Th1 effects of IL-12 in some animals. In other aspects the invention includes a method of inducing a Th1 immune response in a subject by administering to the subject a combination of adjuvants in an effective amount for inducing a Th1 immune response. The formulations of the invention are administered in pharmaceutically acceptable solutions, which may routinely contain pharmaceutically acceptable concentratios of salt, buffering agents, preservatives, compatible carriers, adjuvants, and optionally other therapeutic ingredients.

Preferred routes of administratio include but are not limited to oral, intradermal, intraperitoneal, etc. An injection may be in a bolus or a continuous infusion. For example the pharmaceutical compositions according to the invention are often administered by intramuscular or intradermal injection. A variety of administratio routes are available. The particular mode selected will depend, of course, upon the particular adjuvants or antigen selected, the age and general health status of the subject, the particular condition being treated and the dosage required for therapeutic efficacy. The methods of this invention, generally speaking, may be practiced using any mode of administratio that is medically acceptable, meaning any mode that produces effective levels of an immune response without causing clinically unacceptable adverse effects. Preferred modes of administratio are discussed above.

*The compositions may conveniently be presented in unit dosage form and may be prepared by any of the methods well known in the art of pharmacy. All methods include the step of bringing the compounds into association with* a *carrier, which constitutes one or more accessory ingredients. In general, the compositions are prepared by uniformly and intimately bringing the compounds into association with a liquid carrier, a finely divided solid carrier, or both, and then, if necessary, shaping the product.*

Iridoid glycoside adjuvant a plant based immunomodulator has created a major need for potent vaccine adjuvant, capable of boosting cellular (Th1) immunity but without toxicity. Alum has no effect on cellular immunity, which is desired for newer generatio vaccines.

Iridoid glycoside adjuvant optimal protection against recombinant antigens maintained and improved immune responses with HBsAg and Typhoid vaccine antigen.

Alum has some limitations for use with next generatio recombinant antigens, alum being a poor inducer of Th1 response (cellular response) and induces Th2 based. (Humoral response), is not likely to offer optimal protection.
The present invention explore the use of alternative adjuvant formulation with MDP emulsion in comparison with HBsAg (Hepatitis) and TT (Tetanus Toxiod) it was not highly potent for HBsAg antigen, as iridoid glycoside adjuvant stood out a better alternative adjuvant, with reference to Th1 and Th2 responses.

An ideal adjuvant should reduce the load of antigen with appropriate stability and long shelf life. Iridoid glycoside adjuvant has reduced the load of antigen from (20ug to 15 ug/ml) as well dose of adjuvant (1.5mg alum to 2.5 ug /ml 299A).

When individual compounds PK-I and PK-II are used as adjuvant against OVA (Weak-antigen) the activity profile is less as compared to iridoid glycoside adjuvant, The adjuvant activity in iridoid glycoside adjuvant may be due to synergy of PK-II and PK-II.

### EXAMPLES

**The following examples are given by way of illustratio of the present invention and should not be construed to limit the scope of the present invention**

### Examples

### Isolation of iridoid glycoside adjuvant

iridoid glycoside adjuvantis a standardized mixture of iridoid glycosides (chander, 1992; Li et al, 1998) Preparatio of the material involves extraction with organic solvents and crystallisation. No chromatography operatio is required. Standardisation by HPLC fingerprinting. Its shelf life is determined to be 167,7 weeks.

### EXAMPLE 1

The powdered root of P.Kurroa (100g) is extracted with dichloromethane (500 ml), the extract is rejected. The marc is extracted with 70% aqueous ethanol (500ml) at 20-25°C. centrifuged and supernatant concentrated at 40± 2°C under diminished pressure to 1/4^{th} of its volume and allowed to stand at 20±5°C for 40 h. The separated solid is filtered off and the filtrate is concentrated to dryness at 40±2°C under diminished pressure. The residue is extracted with boiling ethyl acetate (500 ml) and the extract is rejected. The residue is dissolved in hot ethanol (400ml), cooled and diethyl either (100 ml) added till turbidity persists. The turbid solution is allowed to stand at 4°C for 24 h, separated solid is recovered by filtratio, dissolved in 400 ml of dry ethanol (distilled and stored on fused cupric sulphate), decolorized by charcoal treatment and concentrated to 1/4^{th} of its volume, cooled and allowed to stand at 4°c for 24 h .The precipitated solid is separated by filtratio, washed with cold ethanol and dried to get iridoid glycoside adjuvant in the ratio of PKI and PKll is 1:2,

### EXAMPLE 2

**P.Kurroa** root powder (100 g) is extracted with petroleum ether (500 ml, 60-80° C), the extract is rejected. The marc is extracted with 95% aqueous ethanol (500 ml) at 20± 5°C.The ethanolic extract is centrifuged to remove suspended matter and concentrated at 40±2°C under vacuo to 1/4 ^{th} of its original volume and allowed to stand at 20±5°C for 36 h. The separated solid is filtered off and the filtrate is concentrated to dryness at 40±2°C under diminished pressure. The residue is extracted with boiling chloroform (500 ml) and then boiling ethyl acetate (500 ml) and the extracts are discarded, The residue is dissolved in hot ethanol (400 ml), cooled and diethyl either (100 ml) added till turbidity persists. The turbid solution is allowed to stand at 4°c for 24 h; the separated solid is recovered by filtratio, dissolved in dry ethanol (400 ml), decolorized by active charcoal, concentrated to 1/4 ^{th} of its volume and allowed to stand at 4°c for 24 h. The separated solid is filtered out and dried to yield iridoid glycoside adjuvant in the ratio of PK1 and PK2 is 1:2.

### EXAMPLE 3

The root powder (500 g) of P.Kurroa is extracted with dichloroethane while refluxing and the extract is rejected. The marc is extracted with EtOAc while refluxing in a Soxhlet for 20 h. The EtOAc extract is centrifuged to remove suspended matter and concentrated under vacuo to 1/4^{th} of its volume and allowed to stand at 20±5°C for 36 h. The separated solid is filtered off and recrystalise from MeOH , yield iridoid glycoside adjuvantin the ratio of PK1 and PK2 is 1:2.

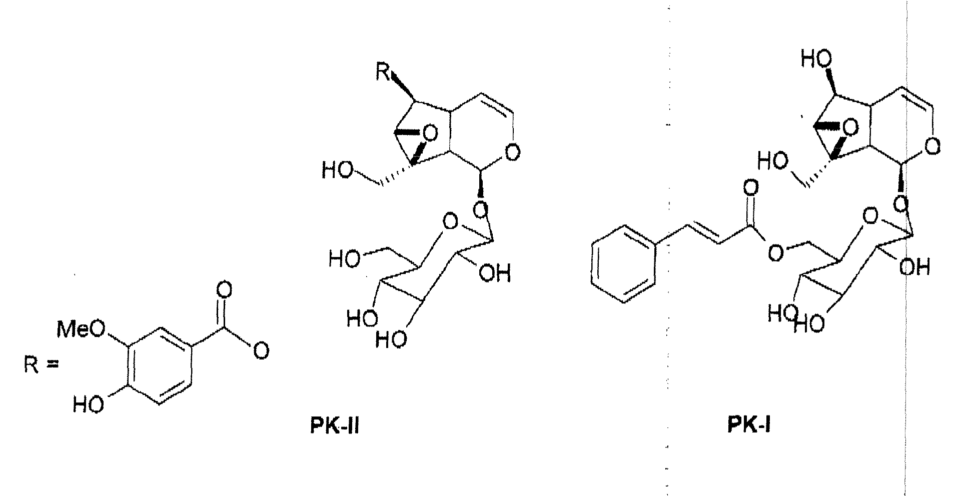

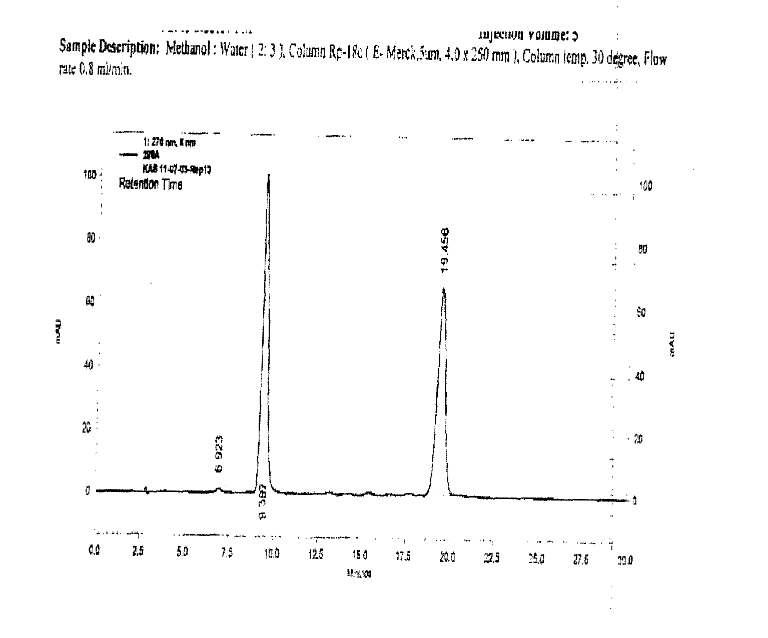

### Comparison of iridoid glycoside adjuvant with recombinant HBsAg antigen

Twelve groups of adult Balb/C mice (each group having 10 mice) were injected with 20 µg HBsAg (i) alone, (ii) mixed with 1.45 mg alum, (iii, iv, v, vi, vii, viii, ix, x) mixed with 0.312, 0.625, 1.25, 2.5, 5, 10, 20, 40 µg. iridoid glycosides from Picrorhiza kurroa or (xi, xii,xiii, xiv, xv, xvi) mixed with variable doses of antigen HBsAg 2.5, 5, 10, 15, 20 and 25 µg. These mice were bled at 15 and 28^{th} day after immunization and the plasma was assayed for anti-HBsAg At the end of the study the mice were killed and their spleens and lymph nodes were removed for assay of CTL activity and cytokines by flow cytometery.

Other groups of mice were immunized with HBsAg (20 µg) alone, with alum (1.45 mg), with variable formulations of adjuvant 0.312, 0.625, 1.25, 2.5, 5,10, 20,40 µg iridoid glycoside adjuvant with both alum and an iridoid glycoside adjuvant.

Other groups of mice (n=10) were immunized as above (except only the 2.5 µg dose of iridoid glycoside adjuvant was used) and boosted with the identical or a different formulation at 8 after two weeks, then spleens were removed 2 weeks later for evaluation of CTL activity.

Groups of Balb/C mice (n=10) were injected with (i, ii, iii) a total of 20µg HBsAg with alum, with iridoid glycoside adjuvant 2.5µg) on with both alum and iridoid glycoside adjuvant or with (iv) an HBsAg alone. Plasma Serum was obtained at 15 & on 28^{th} day for assay of anti-HBsAg as total IgG and IgG subtypes (lgG1 and lgG2a). At the end of the study the mice were killed and their spleens removed for assay of CTL activity.

### Example 4

### Evaluation of Humoral Response to HBsAg

Mice heparinized Blood serum was collected by retro-orbital sinus puncture on day 28 as described elsewhere (Bres et al, 2001). Plasma Serum was recovered by centrifugation (7 min, 13,000 rpm). Antibodies specific to HBsAg in plasma serum were detected and quantified by end-point dilution ELISA assay (in triplicate) on individual samples. Ten-fold serial dilutions of plasma serum were first added to 96-well microtiter plates with a solid phase consisting of plasma -derived HBsAg particles (100 µl/well of HBsAg subtype at 1 µg/ml, coated overnight at RT) and incubated for 1 h at 37° C. The bound antibodies were then detected by incubation for 1 h at 37° C with HRP-conjugated goat anti-mouse IgG, lgG1 or lgG2a (1:400 in PBS-Tween, 10% FCS; 100 µl/well, BD Pharmingen), followed by incubation with OPD solution (100 µl/well, Sigma,) for 30 min at RT room temperature in the dark. The reaction was stopped by the addition of sulphuric acid (50 µl of 4N H₂SO₄). End-point titers were defined as the highest plasma dilution that resulted in an absorbance value (OD at 450) two times greater than that of non-immune plasma Titres were expressed in milli international units per ml (mlU/ml) and absorbance was measured in ELISA reader (High tech Jupiter) at 450 nm.

Sera of immunized mice collected over the course of two immunizations were analyzed by ELISA to determine the induction and duratio of antibody response. Low but detectable serum antibodies specific for HBsAg were found at comparable levels in all mice immunized with alum after the second immunization. However, compared to mice immunized with iridoid glycoside adjuvant 2.5 µg) significantly high antibody levels after second immunization, but iridoid glycoside adjuvant (2.5 µg) plus alum (1.45 mg) showed synergistic effect and produced high antibody levels.
In this study, we also determined by isotyping the effects of adjuvant on induction of different IgG subclasses. Significant differences were indeed observed with the use of alum. It is evident that adjuvant (alum) tested favored IgG1 subclass; however, iridoid glycoside adjuvant was effective in induction of significant levels of IgG2a and IgG1 antibodies suggesting a shift toward both Th1 and Th2 type immune response.

### Example 5

### Immunophenotyping of CD4 & CD8 surface marker by flowcytometery

The spleen (1/3 of the organ) was placed in PBS buffer (without Mg²⁺ and Ca²⁺) stored On ice prior to preparatio of single cell suspensions. Splenic erythrocytes were lysed with red blood cell lysing buffer (BD pharmingen). Cell suspensions were refrigerated (Ca. 4°C) pending staining with antibodies. All reagents were purchased at BD pharmingen. For each sample, 2x 10⁶ cells were stained with conjugated anti-mouse CD4 FITC and anti-mouse CD8a PE antibodies. After staining with antibodies, cells were washed and resuspended in PBS for flow cytometric analysis, which was performed on a FACS Calibur flow cytometer equipped with Cell Quest software (Becton Dickinson). Absolute cell counts per spleen and the corresponding subpopulation cell counts were calculated from the partial weight of the spleen processed for flow cytometry as well as the corresponding cell count and the total spleen weight.

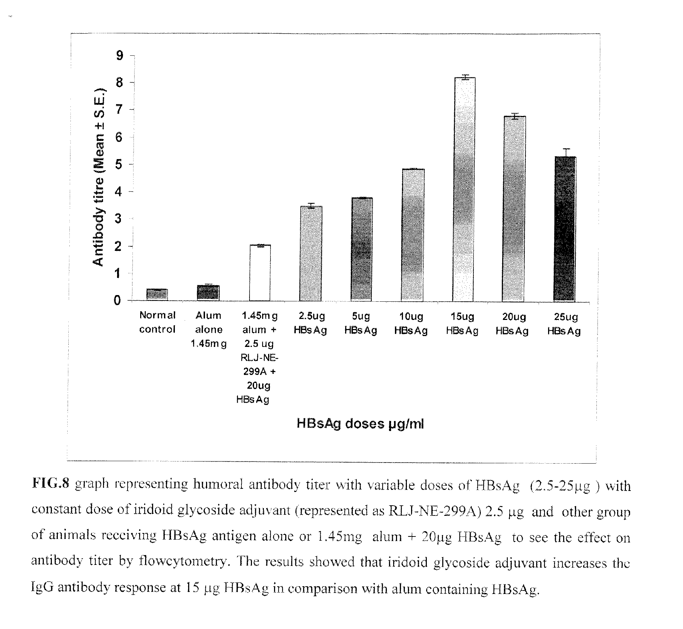

The effect of iridoid glycoside adjuvant on CD8⁺ T cells lymphocytes is shown in figure 8. Compared with the control group, iridoid glycoside adjuvant 2.5 µg) caused a significant increase of CD8⁺ T cells lymphocytes, but there is no significant increase in CD8 T lymphocytes was observed in alum containing HBsAg. But iridoid glycoside adjuvant (2.5 µg) plus alum (1.45 mg) showed synergistic effect and produced significant increased in CD8 population as compared with standard adjuvant alum.

Cytokines (Th1 & Th2 responses) in serum by ELISA (Quantikine, R & D systems) and lymph node cells by flowcytometry collected from immunized mice on day 28^{th} (Quantikine, optia Kit).

Determination of IL-2, IFN gamma, TNF alpha, IL-4, IL-5 and IL-10 in lymph nodes cell culture fluid determined by flowcytometry clearly showed that HBsAg containing iridoid glycoside adjuvant induced both Th1 (IL-2 , IFN-γ and TNF alpha) and Th2 (IL-4, IL-5 and IL-10) cytokines. High levels of Th1 cytokines were secreted in response to MDP and FCA containing HBsAg. Cytokine levels in lymph nodes cell culture fluids produced by cells from mice receiving MDP and FCA containing HBsAg were very low for the Th2 cytokines and increased Th1 type of immune response.

### Example 6

### Mitogen activity

Splenocyte single cell suspension was prepared by up-downing 4m1 RPMI-1640 in spleen and after omitting RBCs using 0.75% NH4C1 in Tris (0.02%, pH=7.2) buffer (adding 6 ml buffer to 2 ml cell suspension after 3 minutes centrifuging at 1000g for 2 min). Concentratio was adjusted to 2x10⁶ cell/ml in RPMI-1640 supplemented by 10% fetal calf serum, 2 mM L-Glutamine, 25 mM HEPES. One hundred microliters of diluted cell suspension were dispensed into 96-well flat bottom culture plate. Mitogen phytohemmaglutinin-A (PHA) was added at 5 µg /ml final concentratio to each well and the volume was adjusted to 0.2 m1. After incubating for 72 h at 37°C and 5% CO₂ humid atmosphere air, cell proliferatio was determined by MTT assay method. Briefly, 10% of (3-(4, 5 diamethyl-2-thiazolyl) 2,5-diphenyl-2H-tetrazolium) (MTT) (5 mg/ml) was added to each well and plates were incubated at 37°C in CO₂ humid atmosphere for 4 h. The blue formazan precipitate was dissolved in acidic Isopropanol and its optical density was measured in 570 nm using Elisa Reader. Each dose was tested in triplicates.

### Example 7

### Toxicity studies:

### Immunization of Rabbits and Guinea pigs with HBsAg and Alum or Alum+iridoid glycoside adjuvant for toxicity studies

### Example 8

### Evaluating the immunogencity of the typhoid antigen withiridoid glycoside adjuvant adjuvant.

Different doses of the adjuvant ranging from 0.3 mcg to 40 mcg were combined with 25 mcg of the Vi polysaccharide antigen. The protocol for evaluating the efficacy of the vaccine was followed as per British Pharmacopiea, wherein, ¼ the of the volume was injected into mice (n=10). A booster dose was given after 14 days. Subsequently after 21 days the animals were bled and the anibody titre was estimated by ELISA techniques, where thyraminated Vi was coated on the plates. Cut off value for antibody generatio was established using the control (saline) value multiplied by 4. All the animals which showed higher values were considered as seroconverted.

Results indicate that combination of 25 µg antigen with 2.5 µg adjuvant showed 100% seroconversion in comparison to other groups. While in case of combination of 2.5 mg adjuvant with 15 mcg of antigen showed 100% seroconversion in comparision to the other groups, which clearly indicate the enhancement of the immune response in the presence of the adjuvant.

### Example 9

Effect of different doses of Ad iridoid glycoside adjuvant on CD4 and CD8 population determined by flowcytometry. Groups of BALB/c mice (*n*=6) were intraperitoneally immunized with 25.0 µg of typhoid antigen alone, Saline as control and associated (typhoid + iridoid glycoside adjuvant with various doses of iridoid glycoside adjuvant: 0.312, 0.625, 1.25, 2.5, 5, 10, 20 and 40. CD4 and CD8 population in splenocytes after the challenging injection were determined by flowcytometry.

Iridoid glycoside adjuvant showed effect of 39.8 % CD4 and 14.8 % of CD8 T cells at 2.5 µg iridoid glycoside adjuvantin 20 µg Ad i.p. dose. The control values were 16.8 % CD4 and 10.6 % for CD8 T cells. This showed a significant increase in CD4/CD8 ratio as shown in Table 1.

**Table 1. Effect of different doses of Ad iridoid glycoside adjuvant on CD4 and CD8 population on splenocytes determined by flowcytometry**

| **Treatment** | **es (mcg)** | **CD4** | **CD8** | **CD4/CD8ratio** |
|---|---|---|---|---|
| Control | - | 16.8±1.24 | 10.6 ±0.88 | 1.58 ±0.33 |
| Commercial | 25 | 24.7±1.86 | 12.8 ± 1.24 | 1.92 ± 0.37 |
| Ad | 0.312 | 20.8±1.2 | 10.6±0.94 | 1.96±0.44 |
| Ad | 0.625 | 20.7 ± 0.84 | 9.8 ± 1.21 | 2.1 ± 0.37 |
| Ad | 1.25 | 26.8 ± 2.41 | 11.8 ±1.43 | 2.27 ± 0.44 |
| Ad | 2.5 | 39.8 ± 3.04 | 14.8 ± 0.89 | 2.68 ± 0.44 |
| Ad | 5 | 30.7 ± 1.24 | 15.8 ± 1.06 | 1.94 ± 0.83 |
| Ad | 10 | 28.6 ± 0.89 | 17.7 ± 0.94 | 1.61 ± 0.44 |
| Ad | 20 | 23.4 ± 2.31 | 15.5 ± 0.88 | 1.50 ± 0.39 |
| Ad | 40 | 20.8 ± 0.67 | 14.7 ± 0.58 | 1.41 ± 0.78 |

Groups of BALB/c mice *(n=6)* were intraperitoneally immunized with 25.0 µg of typhoid antigen alone, Saline as control and associated (typhoid + iridoid glycoside adjuvant with various doses of iridoid glycoside adjuvant: 0.312, 0.625, 1.25, 2.5, 5, 10, 20 and 40. CD4 and CD8 population in splenocytes after the challenging injection were determined by flowcytometry.

The results indicate that 2.5 µg iridoid glycoside adjuvant in 20 µg Ad i.p. dose showed a significant increase in CD4/CD8 ratio on splenocytes.

### Example 10

Effect of test adjuvant Ad iridoid glycoside adjuvant on T (IFN gamma) and B (IL-4) cell secreted cytokines by ELISA, Groups of BALB/c mice (*n*=6) were intraperitoneally immunized with 25.0 µg of typhoid antigen alone, Saline as control and associated (typhoid + iridoid glycoside adjuvant with various doses of iridoid glycoside adjuvant: 0.312, 0.625, 1.25, 2.5, 5, 10, 20 and 40. Splenocytes were collected two weeks after the challenging injection were determined by ELISA.

Splenocytes were isolated from different groups of treated and untreated mice. For cytokine estimation 2×10⁶ cells/ml were cultured with Con A (0.5µg/ml) in 24-well tissue culture plates in RPMI-FBS (10%). Plates were incubated at 37°C in a humidified atmosphere of 5% carbon dioxide for 48h and the supernatants collected for cytokines (IFN-γ and IL-4) assays as per the instructions of the manufacturer (BD OptElA set). Briefly, 96- well micro titer ELISA plates were coated overnight with 50µl of capture antibodies in carbonate- bicarbonate buffer, pH 9.6 at 4° C. After washing, the wells were incubated with 200µl of blocking buffer for 1 h at room temperature followed by addition of 100 µl of supernatant obtained from lymphocytes culture. The plates were kept at room temperature for 2 h, washed five times with wash buffer. Biotin labeled secondary antibody was added along with avidin horseradish peroxidase (AV-HRP). The wells were washed and the substrates, tetramethylbenzidine and hydrogen peroxide were added to each well and incubated at room temperature for the development of color. The reaction was stopped with 1M H3PO4 and plates read at 450 nm. The cytokines were quantified using recommended cytokines standards (BD OptEIA set).

**Table 2. Effect of test adjuvant iridoid glycoside adjuvant (Ad) on T (IFN gamma) and B (IL-4) cell secreted cytokines by ELISA**

| **Treatment** | **Doses (mcg)** | **IFN-γ (pg/ml)** | **IL-4 (pg/ml)** |
|---|---|---|---|
| Control | - | 68.5 ± 3.24 | 88.5 ± 2.64 |
| Commercial | 25 | 349.6 ± 6.83 | 121.3 ± 3.56 |
| Ad | 0.312 | 194.6 ± 7.46 | 96.8 ± 5.21 |
| Ad | 0.625 | 257.4 ± 8.64 | 139.5 ± 7.54 |
| Ad | 1.25 | 358.3 ± 10.4 | 173.5 ± 5.76 |
| Ad | 2.5 | 402.5 ± 7.84 | 214.8 ± 4.21 |
| Ad | 5 | 315.8 ± 5.66 | 191.6 ± 3.58 |
| Ad | 10 | 298.4 ± 7.83 | 159.7 ± 4.34 |
| Ad | 20 | 257.4 ± 8.58 | 120.6 ± 3.38 |
| Ad | 40 | 195.6 ± | 79.6 ± 4.56 |

Groups of BALB/c mice (*n*=6) were intraperitoneally immunized with 25.0 µg of typhoid antigen alone, Saline as control and associated (typhoid + iridoid glycoside adjuvant with various doses of iridoid glycoside adjuvant: 0.312, 0.625, 1.25, 2.5, 5, 10, 20 and 40. Splenocytes were collected two weeks after the challenging injection were determined by ELISA.
The results indicate that iridoid glycoside adjuvant at 2.5 µg increases both Th1 (IFN gamma) and Th2 (IL-4) response in comparison to commercial vaccine, which elicits only Th1 type of the immune response.

### ADVANTAGES

1. Iridoid glycoside adjuvant (2.5ug) with hepatitis B vaccine *in vivo* induced greater than 100 fold anti HBsAg titers than seen with licensed vaccine on alum., so this result is surprising, and thus helpful in generating protective responses in poor responders.
2. Iridoid glycoside adjuvant exhibits potency at microgram quantities and integrates easily with HBsAg formulation.
3. This vaccine formulation having the main HBsAg antigen and immunostimulant iridoid glycoside adjuvant when compared with the alum, MDP and FCA co-administered with HBsAg. shows increased levels of cytokine (Th1 and Th2) where as MDP and FCA induced only Th1 type of immune response. Induced strong and protective immune responses comparatively and reduced the antigenic load.
4. Administratio of iridoid glycoside adjuvant together with HBsAg increases specific IgG1and IqG2a response in mice as compared with alum co-administered with HBsAg, which increases only IgG1 response but poorly elicited IgG2a response.
5. Adjuvant iridoid glycoside adjuvant exhibits potency at microgram quantities in antigen HBsAg but alum exhibits potency at milligrams in antigen HBsAg.
6. Accelerated stability or potency remains stable at three months as compared with alum which is stable only for a month
7. Effect of Adjuvant iridoid glycoside adjuvant together with antigen HBsAg shows no toxic effect as compared with alum, which shows toxic effect
8. The invention is useful in one aspect as a method of inducing an antigen specific immune response.
9. Iridoid glycoside adjuvant formulation is safe, well tolerated and immunogenic and may promote more rapid protection against hepatitis B infection.
10. The composition with iridoid glycoside adjuvant favors an enhancement in the IgG titers immune responses against HBsAg antigen containing alum. The composition comprising iridoid glycoside adjuvant have reduced dose.
11.Iridoid glycoside adjuvant has the potential ability to increase total vaccine HBsAg specific antibody response at 2.5 µg/ml and T cell response.
12. Administratio of iridoid glycoside adjuvant and alum together with HBsAg generates higher protective serum IgG antibody response to that antigen HBsAg present in alum, proving synergetic effect.
13. Iridoid glycoside adjuvant appears to increase potency with relatively small quantities of antigen and exhibit synergy with other adjuvants because of the ability of iridoid glycoside adjuvant to improve the body's immune response to very low doses of antigen.
14. Adjuvant iridoid glycoside adjuvant together with antigen HBsAg reduces the dose of antigen from 20µg (standard vaccine containing 20 µg + alum) to 15 µg (iridoid glycoside adjuvant + HBsAg).
15. Administratio of iridoid glycoside adjuvant together with HBsAg generates better response to increase both the responses Th1 and Th2 as compared with alum co-administered with HBsAg, which enhance only Th2 response.
16. Iridoid glycoside adjuvant together with antigen HBsAg promotes both the population CD4 as well as CD8 population observed on day 15 and 28 in mice as compared with alum co-administered with HBsAg, which is poorly elicited CD8 population enhance only CD4 population.
17. Administratio of iridoid glycoside adjuvant together with HBsAg increases specific IgG1 and lgG2a response in mice as compared with alum co-administered with HBsAg, which increases only lgG1 response but poorly elicited lgG2a response.
18. Adjuvant iridoid glycoside adjuvant exhibits potency at microgram quantities in antigen HBsAg but alum exhibits potency at milligrams in antigen HBsAg.
20. Effect of variable doses of adjuvant iridoid glycoside adjuvant together with antigen HBsAg (20 µg) on serum immunoglobulins, its effect up to 1:4500 dilution on 2.5 µg iridoid glycoside adjuvant in comparison with alum containing HBsAg.
21. Effect of adjuvant iridoid glycoside adjuvant (2.5 µg) together with variable doses of antigen HBsAg on serum immunoglobulins, its effect on 15 µg HBsAg results in significantly influenced antibody response and cell mediated immunity but alum influenced only antibody response but poorly elicited cell mediated immunity.
23. Effect of Adjuvant iridoid glycoside adjuvant (2.5 µg) together with antigen HBsAg (15 µg) enhanced IL-2, IL-12, IFN-gamma, TNF-alpha secreted by Th1 cells and IL-4, IL-5, IL-10 secreted by Th2 cells in comparison with alum, FCA and MDP.
24. Iridoid glycoside adjuvant induces proliferatio of almost all (>92%) B cells and increases immunoglobulin (lg) secretion.
25. The invention is useful in one aspect as a method of inducing an antigen specific immune response.
26. Effect of the immunogenicity of the combination of Vi polysaccharide in combination with iridoid glycoside adjuvant at 2.5 µg adjuvant with 15 µg of the antigen.
27. Effect of the combination for inducing higher titre in compassion to the commercial vaccine.

## Claims

1. An iridoid glycoside composition useful as an adjuvant, said composition comprising picroside I and picroside II represented by following formula, wherein the ratio of picroside I ranges between 0.80 to 1.25 and picroside II ranges in between 1.60 to 2.50 optionally along with other adjuvants. 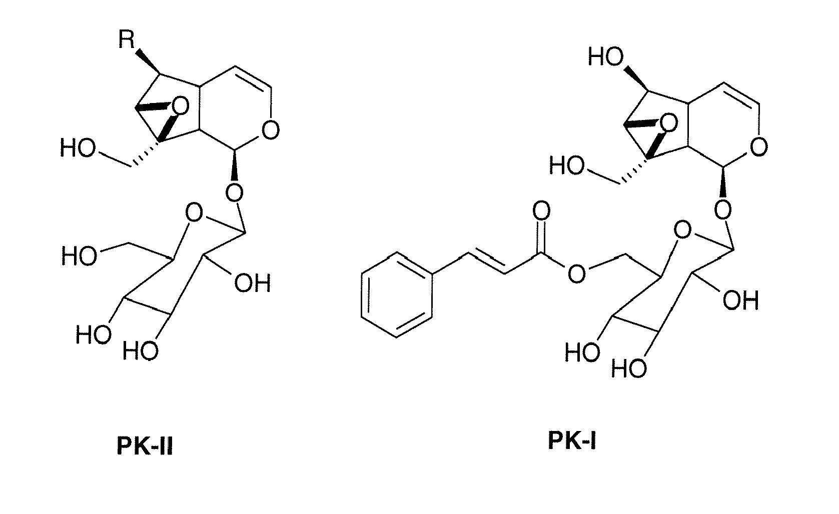 wherein

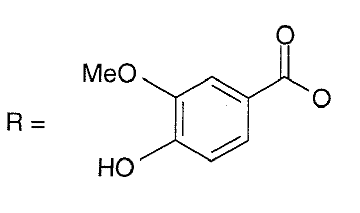

2. An iridoid glycoside composition as claimed in claim 1, wherein the ration of picroside I and picroside II is 1:2.

3. An iridoid glycoside composition as claimed in claim 1 or 2, wherein the other adjuvants are selected from the group comprising alum, monophosphoryl lipid, Complete Freund's adjuvant and Muramyl dipeptide.

4. An iridoid glycoside composition as claimed in anyone of claims 1 to 3, wherein the shelf life of the composition is about 175 weeks.

5. An iridoid glycoside composition as claimed in anyone of claims 1 to 4, wherein the said composition increases levels of both the cytokine Th1 and Th2.

6. An iridoid glycoside composition as claimed in anyone of claims 1 to 5, wherein the composition together with an antigen promotes CD8 population.

7. An iridoid glycoside composition as claimed in anyone of claims 1 to 6, wherein the composition is useful for activation of cells if immune system.

8. An iridoid glycoside composition as claimed in anyone of claims 1 to 7, wherein the effect of variable doses of composition together with antigen HBsAg (20 µg) on serum immunoglobulins, its effect on 2.5 µg bioactive fraction (adjuvant) in comparison with alum containing HBsAg.

9. An iridoid glycoside composition as claimed in anyone of claims 1 to 8, wherein the composition (2.5 µg) together with variable doses of antigen HBsAg on serum immunoglobulins, its effect on 15 µg HBsAg in comparison with alum.

10. A vaccine formulation comprising an iridoid glycoside composition as claimed in anyone of claims 1 to 9 and an antigen optionally along with other adjuvants and pharmaceutically acceptable additives.

11. A vaccine formulation as claimed in claim 10, wherein the antigen is selected from the group comprising peptides, polypeptides, cells, cell extracts, polysaccharides, polysaccharide conjugates, lipids, glycolipids, carbohydrates Virus particles and recombinant proteins, a crude, purified or recombinant form, peptide mimics of polysaccharides, tumor antigen, allergen, bacteria, fungus, protozoa, parasites.

12. A vaccine formulation as claimed in claim 10 or 11, wherein the antigen is HBsAg or typhoid Vi polysaccharide.

13. A vaccine formulation as claimed in anyone of claims 10 to 12, wherein the other adjuvants are selected from de group comprising alum, monophosphoryl lipid, Complete Freund's adjuvant and Muramyl dipeptide.

14. A vaccine formulation as claimed in anyone of claims 10 to 13, wherein the ratio of iridoid glycoside composition, alum and antigen is 0.125:72.5:1.

15. A vaccine formulation as claimed in anyone of claims 10 to 14, wherein the said formulation comprises recombinant hepatitis B surface antigen protein and iridoid glycoside composition optionally along with alum.

16. A vaccine formulation as claimed in claim 15, wherein the said formulation consists iridoid glycoside composition, alum and recombinant hepatitis B surface antigen protein in a ration of 0.125:72.5:1.

17. A vaccine formulation as claimed in anyone of claims 10 to 16, wherein the said formulation consists of iridoid glycoside composition and recombinant hepatitis B surface antigen protein.

18. A vaccine formulation as claimed in claim 17, wherein the ratio of iridoid glycoside ranges between 1 to 128 and recombinant hepatitis B surface antigen protein between 1 to 10.

19. A vaccine formulation as claimed in anyone of claims 10 to 18, wherein the said formulation comprises typhoid Vi polysaccharide and iridoid glycoside composition optionally along with alum.

20. A vaccine formulation as claimed in claim 19, wherein the ratio of iridoid glycoside composition, alum and typhoid Vi polysaccharide is 0.1:58:1.

21. A vaccine formulation as claimed in claims 10 to 20, wherein the said formulation is useful for activation of cells of immune system.

22. A vaccine formulation as claimed in anyone of claims 10 to 20, wherein the said formulation is useful for modulation of cytokine levels.

23. A vaccine formulation as claimed in anyone of claims 10 to 20, wherein the said formulation is useful for modulation of Th1 and Th2 cytokine.

24. Use of an iridoid glycoside composition as an adjuvant, wherein the said composition comprises picroside I in a ratio ranging between 0.80 to 1.25 and picroside II in a ration ranging between 1.60 to 2.50, for manufacturing a composition.

25. A use as claimed in claim 24, wherein the shelf life of the iridoid glycoside composition is about 175 weeks.

26. A use as claimed in claim 24, wherein the iridoid composition increases levels of both the cytokine Th1 and Th2 when coadministered with an antigen.

27. A use as claimed in claim 24, wherein the iridoid glycoside composition is capable of producing a total antigen specific antibody response and T cell response.

28. A use as claimed in claim 24, wherein the iridoid glycoside composition induces antigen specific IgG1 and IgG2 response.

29. A use as claimed in claim 24, wherein the use the iridoid glycoside composition results in enhancement in the IgG titers against antigen containing alum.

30. A use as claimed in claim 24, wherein the use of iridoid glycoside composition together with an antigen promotes CD8 population.

31. A use as claimed in claim 24, wherein the use of the composition together with an antigen enhances IL-2, IL-12, IFN-gamma, TNF-alpha secreted by Th1 cells and IL-4, IL-5, IL-10 secreted by Th2 cells.

32. Use of a vaccine formulation as claimed in anyone of claims 10 to 23 for immunization of a subject with an effective dose of said vaccine formulation.

33. Use as claimed in claim 32, wherein said formulation is administered to a subject by injection.

34. Use as claimed in claim 32, wherein the said formulation is administered by oral, intradermal, intraperitoneal, intramuscular route.

35. Use as claimed in claim 32, wherein the effective dose of said formulation ranges between 0.312 to 40 µg.

36. Use as claimed in claim 32, wherein the said formulation can be injectible as bolus or continuation infusion.

37. Use as claimed in claim 32, wherein the said formulation is administered as unit doses and booster doses.

38. Use as claimed in claim 32, wherein the said subject is a mammal.

39. A process for separation of adjuvant as claimed in anyone of claims 1 to 9, said process comprises the steps of:
a) extracting the powdered dried roots of P. Kurroa with an organic solvent selected form a group consisting of dichloroethane, methane, petroleum ether, and dichloroethane to obtain the marc,
b) extracting the said marc with an organic solvent up to a refluxing temperature,
c) separating the extract form the suspended particles and concentrating to obtain the residue,
d) extracting the residue obtained in step (c) with chloroform and ethyl acetate and extracts are discarded and the residue in dissolved in ethanol or methanol,
e) cooling the ethanolic/methanolic solution obtained in step (d), and adding diethyl ether till turbidity persists,
f) recovering solid by filtration and decolorizing using activated charcoal as decolonizing agent to obtain the mixture of picroside I & II,
g) standardizing the above said mixture of picroside I & II by HPLC fingerprinting,
h) mixing the picroside I and picroside II in a ratio of 1:2 to obtain iridoid glycoside composition.
